# EUROPEAN PATENT SPECIFICATION

(11) **EP 1 421 938 B1**
(45) Date of publication and mention of the grant of the patent: **03.02.2010**
(21) Application number: 02758880.5
(22) Date of filing: 29.08.2002
(51) Int. Cl.: A61K 31/409, A61K 47/48, A61P 35/00, A61P 43/00, C07D 487/22

(54) **ANTITUMOR AGENTS AND PROCESS FOR PRODUCING THE SAME**
ANTITUMORALE MITTEL UND VERFAHREN ZU IHRER HERSTELLUNG
AGENTS ANTICANCEREUX ET LEUR PROCEDE DE PRODUCTION

(30) Priority: 31.08.2001 JP 2001264918
(43) Date of publication of application: 26.05.2004
(73) Proprietor: Maeda, Hiroshi, Kumamoto-shi, Kumamoto 862-0909 (JP)
(72) Inventor: MAEDA, Hiroshi, Kumamoto-shi, Kumamoto 862-0909 (JP); SAWA, Tomohiro, F 69008 Lyon (FR)
(74) Representative: Diehl & Partner
(86) International application number: PCT/JP2002/008707
(87) International publication number: WO 2003/018007

(56) References cited:
- WO-A1-91/18006
- DE-A- 2 645 079
- JP-A- 55 144 028
- US-A- 5 849 259
- DATABASE EMBASE [Online] ELSEVIER SCIENCE PUBLISHERS, AMSTERDAM, NL; 1995, SU B ET AL: "Synthesis of water-soluble derivatives of porphyrin-metal complex and its effects on tumor cell growth" XP002347207 Database accession no. EMB-1996033559 & CHINESE PHARMACEUTICAL JOURNAL 1995 CHINA, vol. 30, no. 12, 1995, pages 746-748, ISSN: 1001-2494
- SAWA T ET AL: "Poly(ethylene glycol) conjugated zinc protoporphyrin: A water-soluble heme oxygenase inhibitor with potent antitumor activity" FREE RADICAL BIOLOGY AND MEDICINE, vol. 31, no. 10, November 2001 (2001-11), page S141, XP002347205 & 8TH ANNUAL MEETING OF THE OXYGEN SOCIETY; RESEARCH TRIANGLE PARK, NORTH CAROLINA, USA; NOVEMBER 15-19, 2001 ISSN: 0891-5849
- DOI K. ET AL.: 'Induction of haem oxygenase-1 by nitric oxide and ischaemia in experimental solid tumours and implications for tumour growth' BR. J. CANCER vol. 80, no. 12, 1999, pages 1945 - 1954, XP002959893
- SAHOO S.K. ET AL.: 'Pegylated zinc protoporphyrin: A water-soluble heme oxygenase inhibitor with tumour-targeting capacity' BIOCONJUGATE CHEM. vol. 13, no. 5, 2002, pages 1031 - 1038, XP002959894
- CHEMICAL ABSTRACTS, vol. 124, 1996, Columbus, Ohio, US; abstract no. 219685Q, SU BINGYIN ET AL.: 'Synthesis of water soluble derivatives of porphyrin metal complex and its effect on tumour cell growth' XP002959898 & ZHONGGUO YAOXUE ZAZHI vol. 30, no. 12, 1995, BEIJING, pages 746 - 748

## Description

### Field of the invention

The present invention relates to anticancer agents with little side effect and excellent tumor accumulation thereby exhibiting a very potent anticancer effect, and the preparation process of the same. More precisely, it relates to anticancer agents containing as the active ingredient heme oxygenase inhibitory metalloporphyrin derivatives that are conjugated with amphipathic or water-soluble polymers, and to a process for preparing them with high efficiency.

### Background technology:

The inventors of the present invention have investigated the relationship between cancer growth or its suppression and the activity of heme oxygenase, and found that heme oxygenase is highly expressed in tumor tissues. The heme oxygenase degrades heme and produces bilverdin, carbon monoxide and free iron in tumor or normal tissues.

Bilverdin is readily converted into bilirubin in the cells, and this bilirubin is a very potent antioxidant. Thereby, bilirubin can be a defense molecule against active oxygen such as peroxide, H₂O₂, or nitric oxide etc. that are generated by leukocytes of the hosts (cancer patients). Namely, bilirubin, thus generated will nullify the toxic oxidative defense power against cancer cells or infecting microbes of the host. Therefore, if one blocks heme oxygenase, no bilirubin will be available and tumor cells will be killed by the oxidative molecules generated by leukocytes as a result of an innate defense state.

The inventors had tried to see the antitumor effect of zinc protoporphyrin (ZnPP), an inhibitor of heme oxygenase, administered into the tumor fading artery of tumor bearing rats thereby targeting the inhibitor into the tumor loci selectively, and they indeed confirmed antitumor effect in rats (K. Doi et al.: Br. J. Cancer 80, 1945-54, 1999).

In Chemical Abstracts, Vol. 124, 1996, Abstract No. 219685 Q, there is disclosed that protoporphyrin was modified with polyethylene glycol, and some metals, like Zn, were introduced into its active centre. The protoporphyrin derivatives inhibited the cell growth of lung metastatic cell lines of murine melanoma.

WO 91/18006 A describes a fluorescent porphyrin used as a marker component in tumour therapy. In this connection, there are mentioned also solubilizing moieties attached to the fluorophore structure. Examples of such solubilizing moieties are polyethylene glycol and derivatives thereof.

From DE 2645079 there are known soluble polymer metal complexes with a metal ion in their centre, the metal being, for example, iron, cobalt, manganese and copper. As an example of the basic structure of the complex there are mentioned porphyrines. The porphyrin may be combined with water soluble polymers like polyethylene glycol..

In British Journal of Cancer, Vol. 80, No. 12 (1999), pages 1945 to 1954, there is described the effect of a haem oxygenase inhibitor treatment on tumour growth by using protoporphyrin.

WO 91/14456 A discloses compositions for photodynamic therapy of cancer cells. There are used porphyrin-type photosensitizers with hydrophilic polymers. A particularly hydrophilic polymer is polyvinyl alcohol, optionally as a copolymer with polyethylene glycol.

EP 0232857 A2 describes a modified haemin linked to polyethylene glycol monomethyl ether through an acid amide bonding.

EP 052723 A2 discloses water-soluble tetraazaporphines wherein the central metal atom is zinc, for example. The compounds may also contain, for example, polyethylene glycol, polyether, polyamine or polyalcohol residues.

EP 0641796 A1 refers to cytotoxic hydro-monobenzoporphyrines to be used for treating non-solid tumour cells. The compounds may contain moieties of polymers, like polyamines, polyethers and polyamine alcohols as well as complexed metal ions like zinc ions.

However, there are several problems to use ZnPP per se as an antitumor agent. Firstly, it is almost insoluble in water per se, thus, one must use an oily formulation to solubilize ZnPP, and such oily formulated agent may be only injectable via the tumor-feeding artery, and this is rather too elaborate and far advanced skill is required for this procedure compared with ordinary intravenous or subcutaneous injection. Secondly, native or original ZnPP has no guarantee for selective accumulation of ZnPP in cancer tissues, and to exert a tumor selective anticancer effect, whereas the drug will be widely distributed to the whole body besides the tumor. Therefore, unexpected side effects are concerned.

On the contrary, the inventors are experts in tumor biology, particularly studied the vascular permeability of solid tumor tissues, and know that macromolecular therapeutics would permeate more selectively at the tumor tissue by virtue of the unique anatomical character and by the effect of multiple vascular permeability factors; and further, those macromolecules are retained in the tumor tissues for long periods.

Thus, this phenomenon was coined "enhanced permeability and retention (EPR)-effect" (Y. Matsumura, H. Maeda: Cancer Res. 47: 6387-92, 1986; H. Maeda: In Advances in Enzyme Regulation (by G. Weber ed), Elsevier Scientific Ltd., Amsterdam, 41, 189-207, 2001).

According to the EPR-effect, drugs with a molecular size larger than 40,000 exhibit a high concentration in blood plasma for a prolonged time, e.g. several hours to days after the intravenous injection, whereas an intratumoral concentration will result in a multiple time, more precisely in 24-48 hr. This means, making the apparent drug size greater than 40,000 would make possible a selective tumor targeting of such macromolecular drugs.

Meanwhile, various metal porphyrin derivatives having inhibitory activity against heme oxygenase, and improved methods of their administrations as a whole were studied. The result is that an amphipathic or water soluble polymer conjugation to the metal protoporphyrins makes it possible to yield water soluble metal porphyrin derivatives and they can be administered not only arterially but also intravenously which has more versatile and easy clinical uses. They exhibit an EPR-effect by polymer conjugation yielding a highly efficient accumulation in tumors, and an enzyme inhibitory activity against heme oxygenase is retained for long periods. As a result, only 2 to 3 times of injections make it possible to suppress tumor growth completely in mice, which is a remarkable result.

Previously, metal porphyrin derivatives possessing heme oxygenase inhibitory activity with an amphipathic or water soluble polymer conjugation were never reported, nor was the method of their preparation before. The inventors have developed the method for synthesis of a certain amphipathic or water-soluble polymer conjugation of metalloporphyrin via an amide linkage as specified in the claims. The resulting polymer conjugated metalloporphyrin derivatives are novel compounds and were not reported previously.

### Disclosure of the Invention

The present invention is directed to anticancer agents containing as the active ingredient metalloporphyrin derivatives having an inhibitory activity against heme oxygenase, especially Zn-protoporphyrin (ZnPP) conjugated with amphipathic polymers which are both water and lipid soluble or water soluble polymers as specified in the claims.

The present invention is also directed to a series of novel useful compounds as ingredients of anticancer agents where amphipathic or water-soluble polymers and heme oxygenase inhibitory metalloporphyrin derivatives are conjugated via amide bonds, and to a preparation process of the compounds as specified in the claims.

### Brief Explanations of drawings

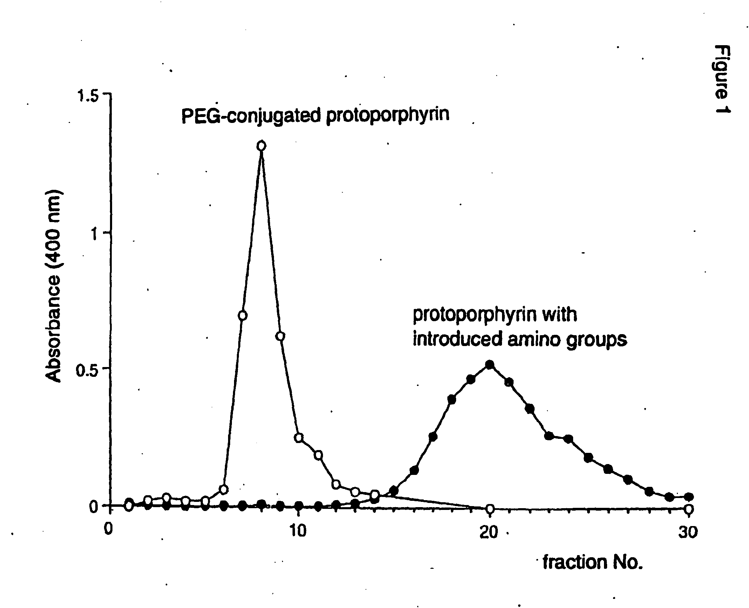

Figure 1 shows the gel filtration chromatography of diaminoethane coupled protoporphyrin and PEG-conjugated protoporphyrin.
Figure 2 shows a Lineweaver-Burk plot of PEG-ZnPP inhibitory profile against heme oxygenase.
Figure 3 shows flow-cytometric analysis data where PEG-ZnPP treated cultured cancer cells exhibit a more oxidant exposured profile.
Figure 4 shows an antitumor effect of PDG-ZnPP in a mouse model with a solid tumor.
Figure 5 shows the profile of body weight change during or after intravenous administration of PEG-ZnPP.

### Most preferable embodiment for carrying out the invention

Amphipathic or water soluble polymers to be conjugated include polyethylene glycol (PEG), polypropylene glycol (PPG), polyvinyl pyrrolidone (PVP), polyvinyl alcohol (PVA), various gelatins, and- their derivatives such as succinylated forms, polyamino acids (eg. polymerized aspartic acid, glutamic acid, lysine, alanine, glycine, proline, tyrosine, etc.), hydroxypropyl and other alkyl acrylate polymers, styrene maleic acid copolymers(SMA), and their derivatives. Among these polymers with amphipathic and water-soluble characters, PEG and SMA are more preferable. PEG with molecular weight of 2000-5000 is preferably used.

SMA is a copolymer of styrene and maleic acid in the alternative order where the carboxyl group of maleic acid can be utilized to conjugate with metalloporphyrin directly or indirectly. SMA can be used as such or as its derivatives where maleic acid is partially esterified.

The metal porphyrin derivative is a complex porphyrin compound, where the metal is chelated in stable coordination to the porphyrin ring, and the protoporphyrin is preferably used because of its easy availability among porphyrin compounds.

Among the metals to be coordinated, iron that gives no heme oxygenase inhibitory action, mercury with a poisonous nature, and monovalent metals which do not form a coordinated chelation, can not be used. However, various metals other than the above such as zinc, tin, cobalt, and copper can be used. Among them, tin and zinc complexes are more preferred. However, tin is also known to be poisonous. Thus, ZnPP is most preferable and its chemical structure is shown in formula B.

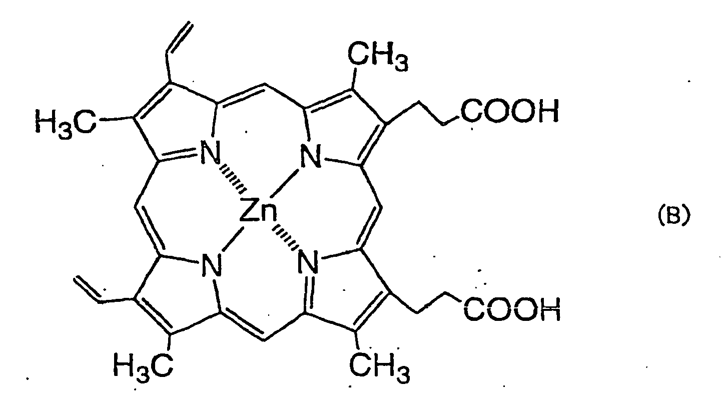

The anticancer agents of the present invention are any macromolecular compounds obtained by conjugation of a metal porphyrin with amphipathic or water-soluble polymers as specified in the claims. However, we found it difficult to carry out the conjugation of the polymers to the metal porphyrin directly, because a metal porphyrin derivative is water-insoluble. To undertake this chemical conjugation, it is preferable to conjugate the polymers to porphyrin before the coordination of metal, and then to coordinate the metal.

The conjugation of porphyrin with the polymer can be facilitated by introducing a desired functional spacer group.

For example, in the synthesis of ZnPP, the polymer can be directly conjugated to the two carboxyl groups in the protoporphyrin, but this direct conjugation method is not advantageous because of the poor activity of said carboxyl groups for this reaction. The inventors of the present invention studied effective synthesis methods of PEG conjugated ZnPP, and succeeded to synthesize ZnPP conjugated with PEG via an amide bond (formula A).

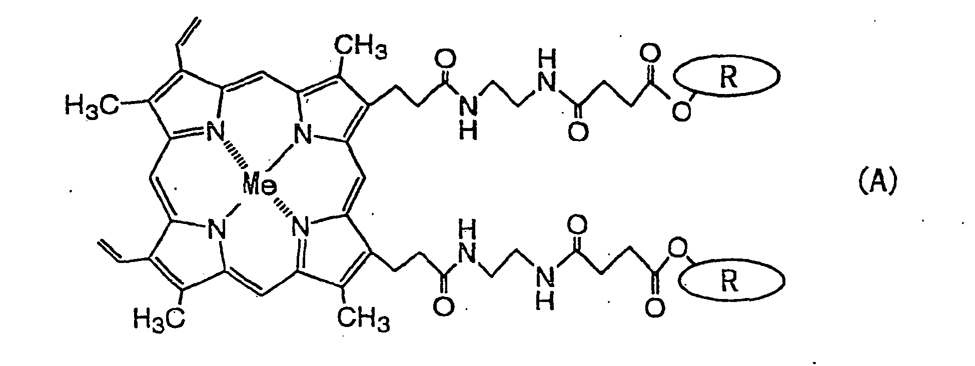

Therein, R means an amphipathic or water-soluble polymer, and Me is a metal.

The polymer conjugated ZnPP B may be synthesized by a successive reaction as follows:
(1)Introduction of the amino group to protoporphyrin IX;
(2)Conjugation of the polymer, and lastly
(3)Coordination of Zn into the porphyrin ring.

For example, the scheme of synthesis of PEG conjugated ZnPP is shown diagrametrically by stepwise reactions as follows.
[Reaction (a)] Protoporphyrin IX (compound (1)) is activated with ethyl chloroformate in tetrahydrofuran (compound (2)).
[Reaction (b)] Protoporphyrin with diamino group (compound (3)) can be obtained by addition of ethylene diamine.
[Reaction (c)] PEG is introduced into the protoporphyrin ring by the addition of activated PEG (compound (4)).
[Reaction (d)]. Lastly, PEG-ZnPP (compound (5)) is obtained by addition of zinc acetate to the reaction product of the Reaction (d). One can replace Zn for tin (Sn) and obtain PEG-Sn-PP by the addition of tin acetate.

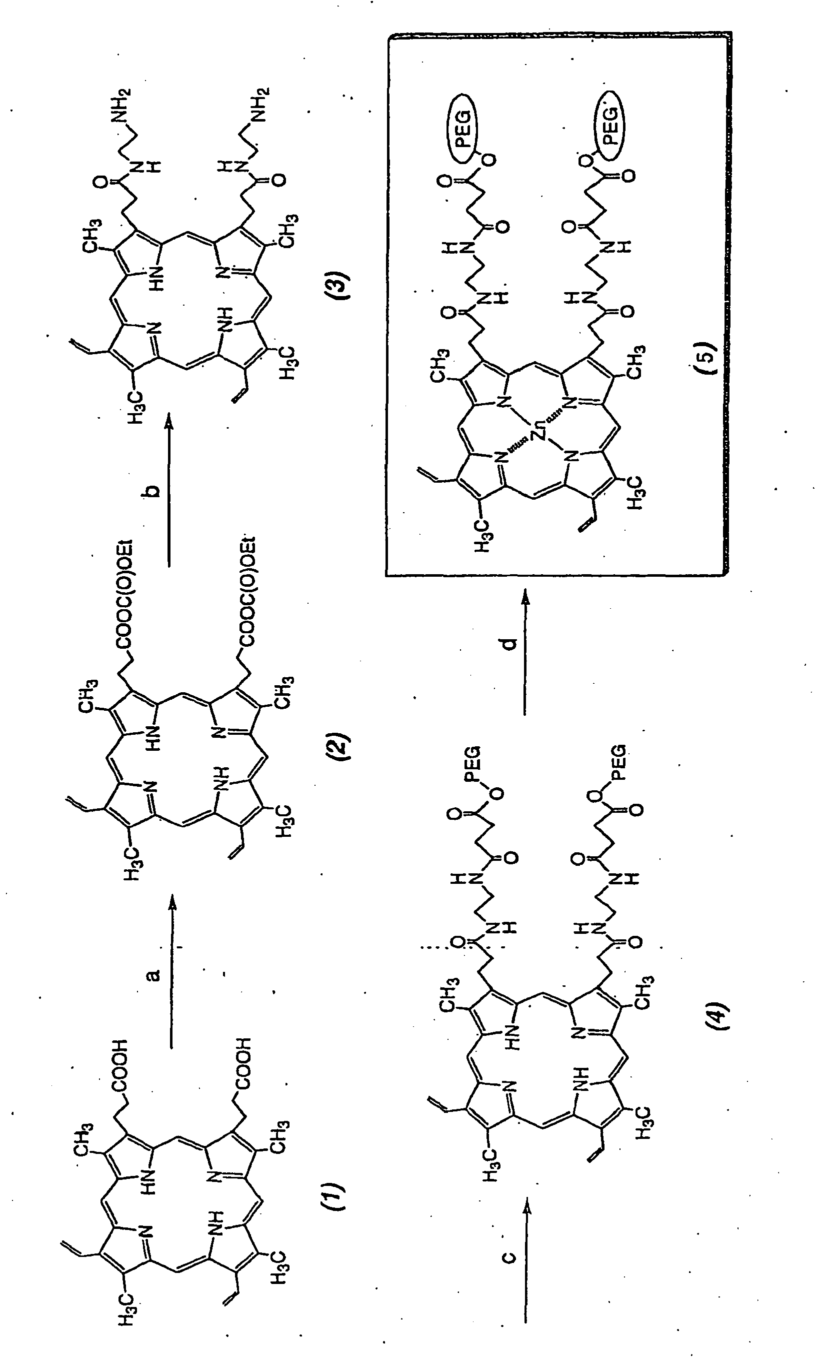

Other compounds than PEG, with amphipathic or water soluble polymers, such as SMA, can be attached to protoporphyrin similarly by condensation reaction of compound (3) and SMA.

The heme oxygenase inhibitory metalloprotoporphyrin, that is conjugated with amphipathic or water-soluble polymers shown in formula A, is selectively accumulated in solid tumors and exhibits excellent antitumor activity. Thus it is a novel and useful antitumor substance. The compound (5), which is a typical example of the compound A, wherein metal is zinc and R is PEG, was synthesized by the scheme (a) to (d). The chemical structure of the reaction product was confirmed by following analysis.

Firstly, the evidence of the amino group of ethylene diamine that was introduced into protoporphyrin (compound (3)) is confirmed by the following:
(1)Infrared spectra with absorbance at 1641cm⁻¹ and 1552cm⁻¹ showed a new formation of the amide bond in the compound (structure of compound (3)).
(2)Determination of molecular weight of the compound by mass spectroscopy (MS) showed 646, identical to the value calculated by the formula based on the compound (3).

Then, PEG (MW about 5000) was coupled to the amino group introduced into the protoporphyrin (compound (3)), and zinc is chelated. The structure of thus obtained ZnPP was identified by determination of the molecular weight and absorption spectra (UV/Vis).

The determination of the molecular weight showed a mass of near 11,000 Da by TOF/MS (time of flight-mass spectroscopy). The UV absorption showed a max. peak at 425, 543, and 583 indicating formula (5) to be PEG-ZnPP.

The scheme of the PEG-ZnPP synthesis using protoporphyrin IX as the starting material via reaction steps [a] - [d] is a novel manufacturing method.

The obtained polymer conjugated metalloporphyrin is readily water-soluble and it may be used as a injection solution either intravenously or arterially

### [Examples]

The process for preparing the PEG-ZnPP, the inhibitory activity of the PEG-ZnPP towards heme oxygenase, and the anticancer effect of PEG-ZnPP by intravenous injection according to the present invention shall be explained in detail with the following examples.

### [Example of Manufacturing]: Synthesis of polyethylene glycol conjugated ZnPP (PEG-ZnPP)

100mg of protoporphyrin IX was dissolved in 20 ml of tetrahydrofuran, and 2.45 ml of triethylamine was added to this solution. This solution was kept at about 0°C on ice, then 1.7 ml of ethyl chloroformate was added to this dropwise under stirring, and allowed to react further for two hours. Subsequently, triethylamine HCl salt being formed was removed by filtration, and 1.2 ml ethylene diamine was added, and the reaction was continued at room temperature for 24 hours. The reaction mixture was then subjected to vacuum evaporation to remove tetrahydrofuran, and the solid material obtained was washed 7 times with 50ml of distilled water yielding 60 mg of porphyrin derivative having two amino groups per molecule (reaction a and b).

Five mg of compound (3) were dissolved in 25 ml of chloroform, and 800 mg of succinimidoester of polyethylene glycol (Shearwater; PEG, MW5000) was added to this solution, and reacted for 24 hours under stirring at room temperature[reaction c].

PEG-conjugated protoporpyrin thus obtained was subjected to gel filtration chromatography on Sephadex LH60 using chloroform as eluent. The result of the gel filtration chromatography showed that unreacted aminated compound (3) did not exist in the preparation of PEG-conjugated protoporpyrin at all. It showed that all aminated protoporphyrin reacted with PEG to form the polymeric form of protoporphyrin. Unmodified protoporphyrin, if any, was eluted at fraction No. 20, where the elution volume was similar to the aminated protoporphyrin.

40mg of zinc acetate was added to the PEG-PP solution and allowed for two hours at room temperature yielding PEG-conjugated zinc protoporphyrin (PEG-Zn-PP) (reaction d).

### [Experimental Example 1]: Inhibitory activity of PEG-ZnPP against heme oxidase.

This was examined using purified heme oxygenase fraction derived from rat spleen. It was assayed at 37 °C in the presence of hemin, the substrate of heme oxygenase, cofactor (NADPH, nicotine adenine dinucleotide), and cytosolic fraction containing bilirubin reductase, in which biliverdin formed by the oxygenase is converted to bilirubin.

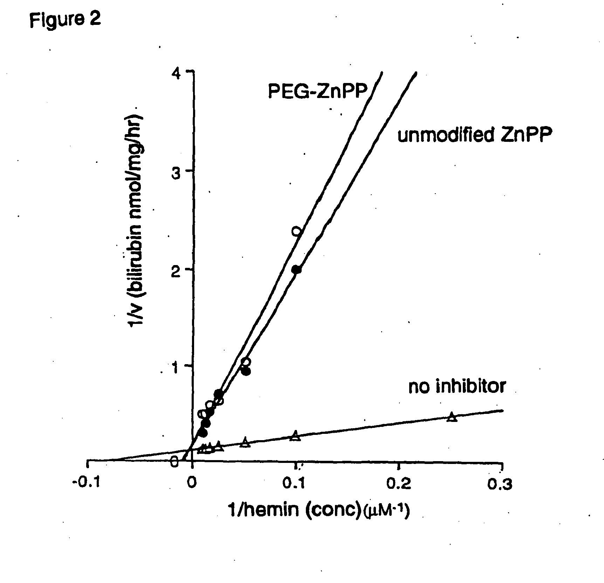

Bilirubin was extracted with chloroform and quantified by absorption at 465 nm. By the addition of either PEG-ZnPP, or unmodified ZnPP, or no inhibitor, their effect on heme oxidase was examined, and the Lineweaver-Burk plot of heme oxygenase activity was plotted during the inhibition by PEG-ZnPP. The result is shown in Figure 2, indicating that PEG-ZnPP inhibits the heme oxygenase in a dose dependent manner, and the inhibitory constant (Ki) was 0.13 µM. The mode of inhibition was competitive, and the value was equivalent to that of unmodified ZnPP (Ki = 0.12 µM).

### [Experimental Example 2]: Effect of PEG-ZnPP on cultured tumor cells.

Lung adenocarcinoma cell line A549 cells were plated in a plastic dish and after an overnight culture, 5 µM and 10 µM of PEG-ZnPP dissolved in distilled water were added to the culture dishes. Then, 8 hours after cultivation at 37 °C, a reagent that quantifies oxidative stress, called dichlorodihydrofluorescein diacetyl ester (DCDHF), was added and followed by cell culture for 30 minutes. Under oxidative stress, this DCDHF will become oxidized and will fluoresce due to oxystress generated by formation of fluorescein in cells.

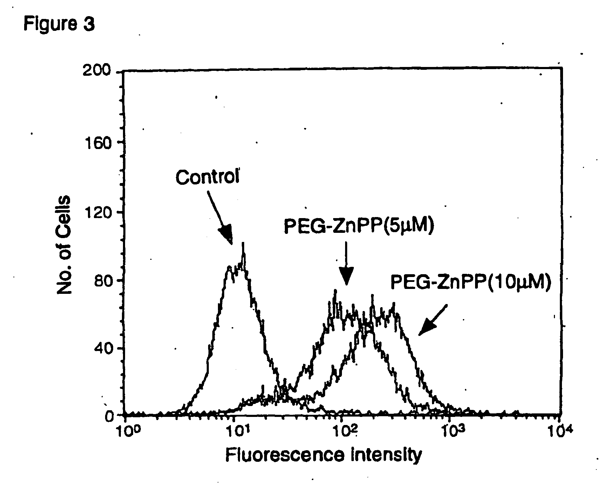

Quantification of fluorescence intensity represents the extent of oxidative stress induced in the cells. Then, cultured cells were trypsinized, and the recovered cells were subjected to the flow cytometrory analysis, and the fluorescence cell population was quantified. The results are shown in Figure 3, where the effect of PEG-ZnPP at 5 µM, 10 µM, is compared with that of no drug. It is clear from these data in Fig. 3 that PEG-ZnPP brought about a higher intracellular oxidative state in the dose dependent manner of PEG-ZnPP.

### [Experimental Example 3]: Inhibition of heme oxygenase in solid tumor model in mouse.

In male ddY mice with a mean body weight of 35 g, S180 sarcoma cells were implanted in the dorsal skin, and when the solid tumor size becomes 5 mm in the cross diameter after about one week, PEG-ZnPP dissolved in distilled water was injected via the tail vein (i.v.) at 0.5 mg ZnPP equivalent per Kg body weight. The solid tumors were removed after 24 hr, and the heme oxygenase activity was quantified similarly as described in Example 1. A control mouse received distilled water without PEG-ZnPP. The tumor specimens where obtained and treated similarly. As shown in Table 1, PEG-ZnPP given i.v. (tail) showed a significant reduction of the heme oxygenase activity. Unmodified ZnPP could not be administered i.v. because of its difficulty in solubility.

**[Table 1]**

| Inhibition of intratumor heme oxygenase by PEG-ZnPP given via the tail vein. | | |
|---|---|---|
| Drug | Activity of heme oxygenase in tumour tissue. | |
| | (n mol bilirubin/mg protein/hr) | |
| Control, none | 4.17 ±1.07 | (P < 0.02) |
| Group of PEG-ZnPP administered | 2.30 ±0.54 | |
| Unmodified ZnPP administered | Impossible to solubilize in water (can not be injected) | |

### [Experiment 4]: Antitumor effect of PEG-ZnPP and change of body weight in mice bearing solid tumor.

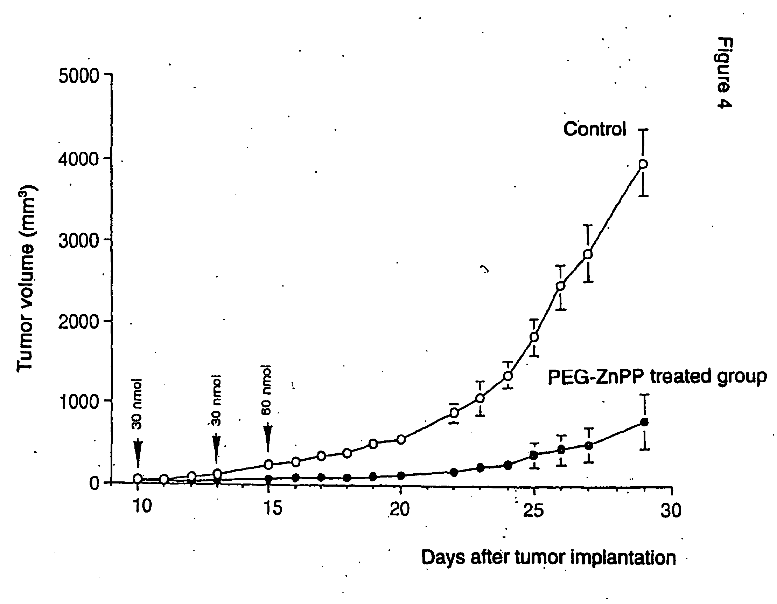

Similar to Experiment 3 above, with sarcoma S 180 of mice implanted under the dosal skin of ddY mice, and after 10, 13 and 15 days after the tumor implantation, PEG-ZnPP at 30 n mole, 30 n mole and 50 n mole (3 times only), respectively, was injected into the tail vein respectively (see also arrow marks in Figure 4). Control mice received distilled water instead of PEG-ZnPP. The sizes of the tumors were measured every week day as shown in Figure 4. It is clear that the PEG-ZnPP group showed a remarkable suppression of tumor growth compared with the control group.

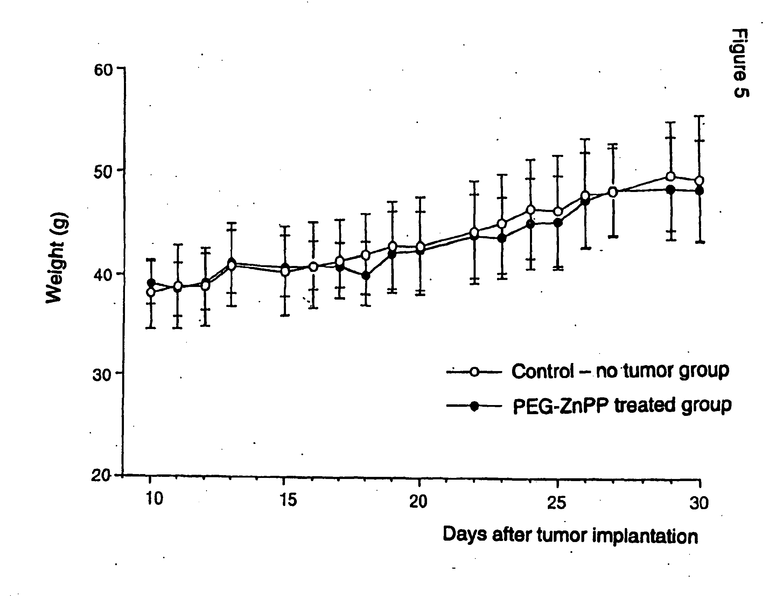

The body weight of both the treated and the non-treated mice was measured simultaneously as seen in Figure 5. There was no remarkable body weight loss in the group treated with PEG-ZnPP.

### Applicability of the Invention in the Industrial Sense

According to the present invention, metal porphyrin derivatives which are inhibitory against heme oxygenase, can be made both water-soluble and lipid soluble. They make the derivatives to become an intravenously injectable medicament by conjugation with amphipathic or water-soluble polymers. This is a novel medicament having an excellent tumor selective accumulation. An effective preparation method of this compound was also found.

The anticancer agents according to the present invention have an excellent anticancer effect without generating any appreciable side effect or toxicity.

Thus the polymer conjugated anticancer agents according to the present invention are highly useful drugs having an excellent tumor selective targeting property with a new mode of action different from many of the known low molecular weight anticancer drugs.

## Claims

1. Anticancer agent comprising as the active ingredient a heme oxygenase inhibitory metalloporphyrin derivative which contains a metal atom chelated to the porphyrin and which is modified by a polymer, **characterized in that** the modifying polymer is a styrene-maleic acid copolymer.

2. Anticancer agent containing as the active ingredient a heme oxygenase inhibitory metalloporphyrin derivative which contains a metal atom chelated to the porphyrin and which is modified by a polymer, **characterized by** general formule. (A) 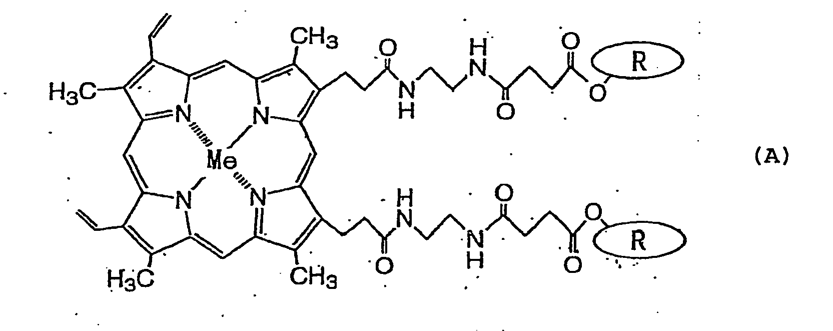 wherein each R means an amphipathic or water-soluble polymer and Me means a metal atom.

3. Anticancer agent according to claim 1 or 2, **characterized in that** the metal atom is zinc or tin.

4. Process for preparing a heme oxygenase inhibitory metalloporphyrin derivative as defined in claim 3, **characterized by** the following steps:
(a) Activating a protoporphyrin of formula (1), which is Protoporphyrin IX, 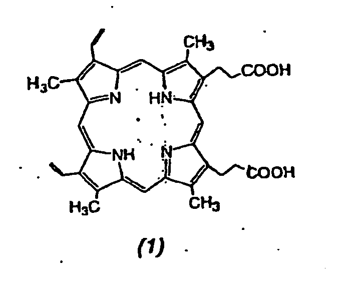 with ethyl chloroformate in tetrahydrofuran to obtain the activated protoporphyrin of formula (2) 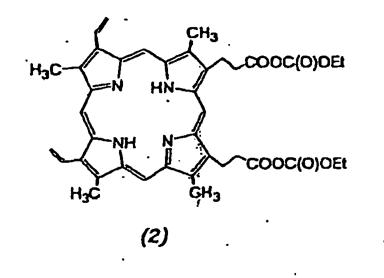 wherein Et means the ethyl group,
(b) adding ethylene diamine to the compound of formula (2) to obtain a protoporphyrin of formula (3), 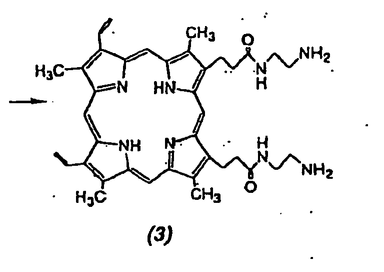
(c) introducing polyethylene glycol into the protoporphyrin ring by the addition of activated polyethylene glycol to then protoporphyrin of formula (3) to obtain a compound of formula (4), 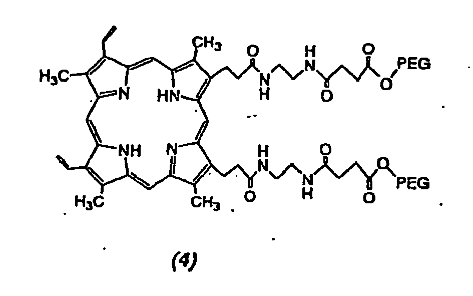 wherein PEG means a polyethylene glycol moiety, and
(d) adding zinc acetate to the compound of formula (4) to obtain the compound of formula (5) 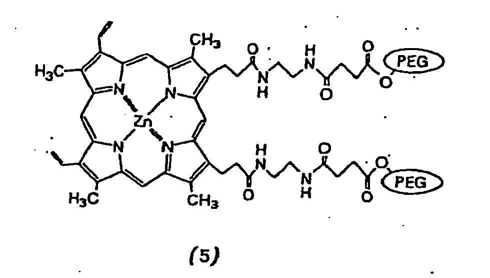 wherein PEG is defined as above.

## Patentansprüche

1. Antitumorales Mittel, als aktiven Bestandteil ein Häm-Oxygenase-hemmendes Metalloporphyrin-Derivat beinhaltend, welches ein an das Porphyrin chelatisiertes Metallatom umfasst, und welches durch ein Polymer modifiziert ist, **dadurch gekennzeichnet, dass** das modifizierende Polymer ein Styren-Maleinsäure-Copolymer ist.

2. Antitumorales Mittel, als aktiven Bestandteil ein Häm-Oxygenase-hemmendes Metalloporphyrin-Derivat beinhaltend, welches ein an das Porphyrin chelatisiertes Metallatom umfasst, und welches durch ein Polymer modifiziert ist, **gekennzeichnet durch** die allgemeine Formel (A) 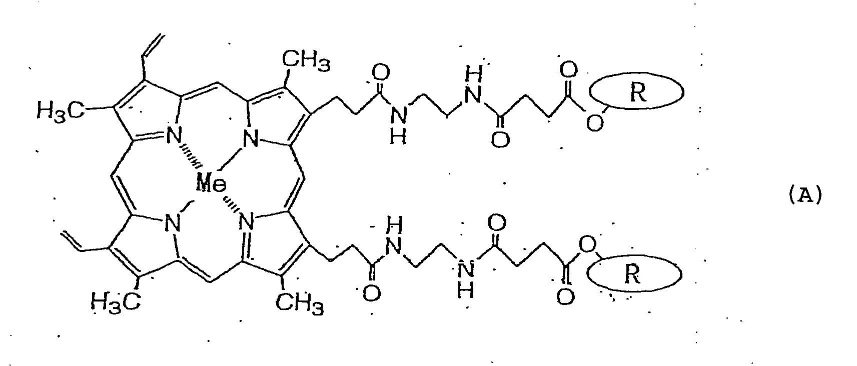 wobei jedes R ein amphipathisches oder wasserlösliches Polymer bedeutet, und Me ein Metallatom bedeutet.

3. Antitumorales Mittel gemäß Anspruch 1 oder 2, **dadurch gekennzeichnet, dass** das Metallatom Zink oder Zinn ist.

4. Verfahren zur Herstellung eines Häm-Oxygenasehemmenden Metalloporphyrin-Derivats gemäß Anspruch 3, **gekennzeichnet durch** die folgenden Schritte:
(a) Aktivieren eines Protoporphyrins der Formel (1), welches Protoporphyrin IX ist, 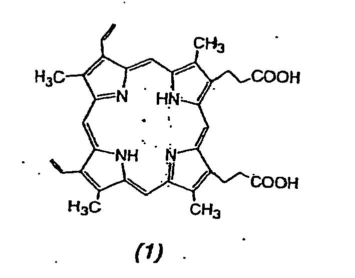 mit Ethylchlorformiat in Tetrahydrofuran, um das aktivierte Protoporphyrin der Formel (2) zu erhalten 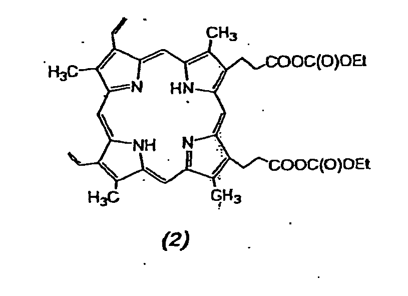 wobei Et die Ethylgruppe bedeutet,
(b) Zufügen von Ethylendiamin zu der Verbindung der Formel (2), um ein Protoporphyrin der Formel (3) zu erhalten, 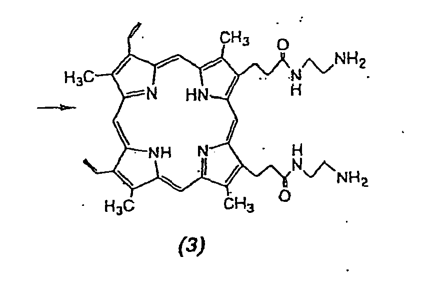
(c) Einfügen von Polyethylenglykol in den Protoporphyrinring **durch** das Zufügen von aktiviertem Polyethylenglykol zu dem Protoporphyrin der Formel (3), um eine Verbindung der Formel (4) zu erhalten, 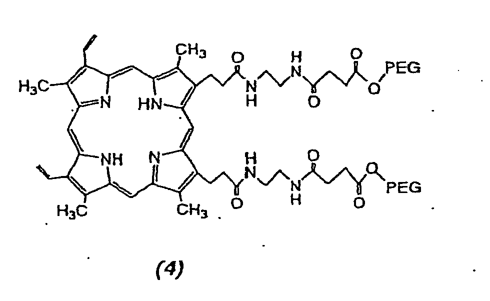 wobei PEG eine Polyethylenglykol-Einheit bedeutet, und
(d) Zufügen von Zinkacetat zu der Verbindung der Formel (4), um die Verbindung der Formel (5) zu erhalten 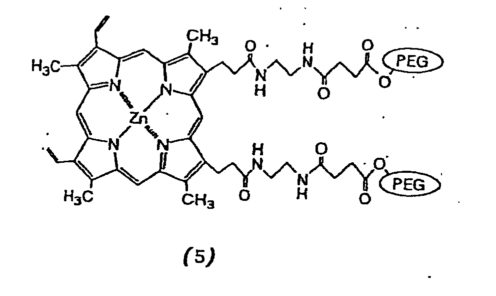 wobei PEG wie oben definiert ist.

## Revendications

1. Agent anticancéreux comprenant en tant que principe actif un dérivé de métalloporphyrine inhibiteur de l'hème oxygénase qui contient un atome métallique chélaté à la porphyrine et qui est modifié par un polymère, **caractérisé en ce que** le polymère modificateur est un copolymère de l'acide styrène-maléique.

2. Agent anticancéreux comprenant en tant que principe actif un dérivé de métalloporphyrine inhibiteur de l'hème oxygénase qui contient un atome métallique chélaté à la porphyrine et qui est modifié par un polymère, **caractérisé par** la formule générale (A) 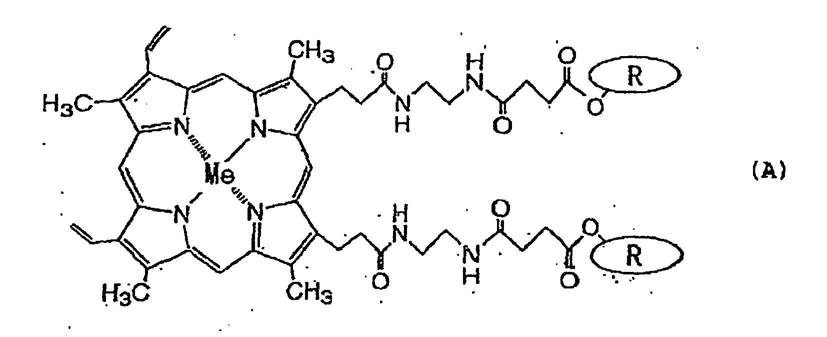 dans laquelle chaque R signifie un polymère amphipathique ou hydrosoluble et Me signifie un atome métallique.

3. Agent anticancéreux selon la revendication 1 ou 2, **caractérisé en ce que** l'ion métallique est du zinc ou de l'étain.

4. Procédé de préparation d'un dérivé de métalloporphyrine inhibiteur de l'hème oxygénase tel que défini dans la revendication 3, **caractérisé par** les étapes suivantes :
(a) activation d'une protoporphyrine de formule (1), qui est la protoporphyrine IX, 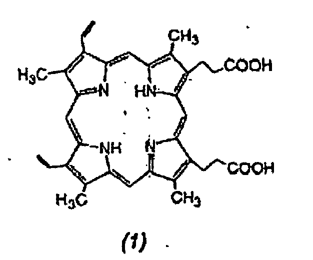 avec du chloroformiate d'éthyle dans du tétrahydrofurane pour obtenir la protoporphyrine activée de formule (2) 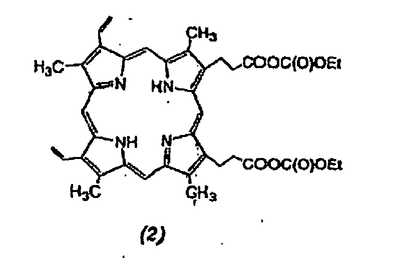 dans laquelle Et signifie le groupe éthyle,
(b) addition d'éthylènediamine au composé de formule (2) pour obtenir une protoporphyrine de formule (3), 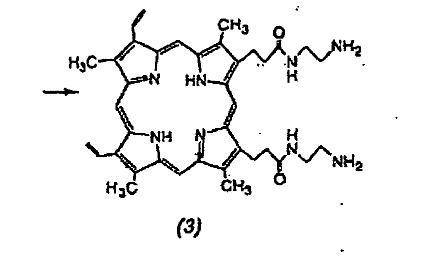
(c) introduction de polyéthylène glycol dans le cycle de protoporphyrine par l'addition de polyéthylène glycol activé à la protoporphyrine de formule (3) pour obtenir un composé de formule (4), 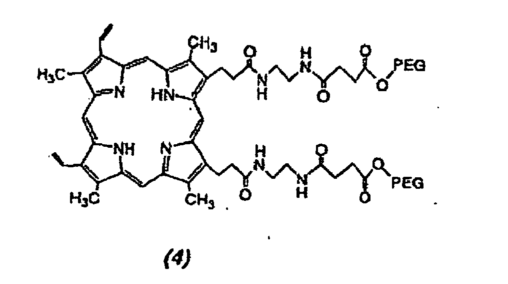 dans laquelle PEG signifie un radical de polyéthylène glycol, et
(d) addition d'acétate de zinc au composé de formule (4) pour obtenir le composé de formule (5) 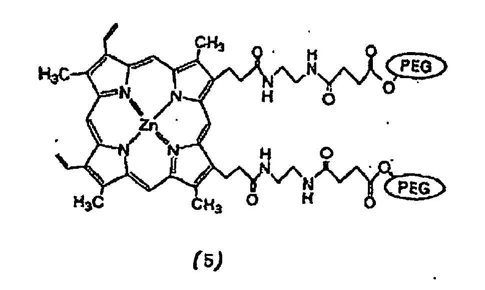 dans laquelle PEG est défini comme ci-dessus.
